# EUROPÄISCHE PATENTSCHRIFT

(11) **EP 4 061 597 B1**
(45) Veröffentlichungstag und Bekanntmachung des Hinweises auf die Patenterteilung: **02.08.2023**
(21) Anmeldenummer: 20801180.9
(22) Anmeldetag: 30.10.2020
(51) Int. Cl.: B29C 33/38, B33Y 99/00, B29C 64/30

(54) **VERFAHREN ZUR HERSTELLUNG EINES IMPLANTATS AUS EINEM BIOKOMPATIBLEN SILIKON**
METHOD FOR PRODUCING AN IMPLANT FROM A BIOCOMPATIBLE SILICONE
PROCÉDÉ DE PRODUCTION D'IMPLANT À PARTIR DE SILICONE BIOCOMPATIBLE

(30) Priorität: 22.11.2019 DE 102019131618
(43) Veröffentlichungstag der Anmeldung: 28.09.2022
(73) Patentinhaber: Novatech SA, 13705 La Ciotat Cedex (FR)
(72) Erfinder: KLEIN, Frank, 14089 Berlin (DE)
(74) Vertreter: Griepenstroh, Jörg
(86) Internationale Anmeldenummer: PCT/EP2020/080532
(87) Internationale Veröffentlichungsnummer: WO 2021/099091

(56) Entgegenhaltungen:
- WO-A2-2004/026178
- CN-A- 105 616 043
- CN-A- 107 296 669
- US-A1- 2006 204 699

## Beschreibung

Die Erfindung betrifft ein Verfahren zur Herstellung eines Implantats aus einem biokompatiblen Silikon gemäß den Merkmalen in Patentanspruch 1.

Je nach Anwendung kann die Verweildauer eines Implantats im Körper wenige Stunden bis lebenslang betragen. Da ein Implantat wie z.B. ein Stent je nach Verwendung in direktem Kontakt mit Körpergewebe, Haut, Schleimhäuten oder Körperflüssigkeiten wie Blut, Speichel oder Sekreten steht, ist es wichtig, dass er aus einem geeigneten Material besteht. Die Eignung für den entsprechenden Anwendungsfall wird auf Basis der DIN EN ISO 10993 beurteilt.

Die Norm ISO 10993 ist eine ISO-Normenreihe zur biologischen Beurteilung von Medizinprodukten. Ziel der Norm ist es die biologische Beurteilung hinsichtlich der Verträglichkeit der eingesetzten Materialien mit dem Körper zu bewerten. Es werden damit nicht nur Produkte, sondern auch Ausgangsstoffe zur Herstellung von Medizinprodukten untersucht. Außer der biologischen Prüfung beinhaltet die Norm zusätzlich physikalisch-chemische Prüfungen und Analysen von gelösten Stoffen und Substanzen und schreibt das Einhalten von Grenzwerten bei herauslösbaren Substanzen vor.

Es ist bekannt, dass eine wesentliche Eigenschaft von Silikonelastomeren die sehr gute biologische Verträglichkeit beim Menschen ist. Silikone bezeichnen eine Gruppe synthetischer Polymere, bei denen Siliziumatome über Sauerstoffatome zu Molekülketten und/oder netzartig verknüpft sind. Die restlichen freien Valenzelektronen des Siliziums sind dabei durch Kohlenwasserstoffreste (meist Methylgruppen) abgesättigt. In der wissenschaftlichen Literatur finden sich statt Silikone häufig die Begriffe Poly(organo)siloxane oder kurz Siloxane.

In der Medizintechnik werden hauptsächlich platinkatalysierte 2-komponentige warmhärtende Silikone verwendet. Diese unterliegen einer strengen Qualitätssicherung und werden als "unrestricted" für die zeitlich unbegrenzte Verweildauer im Patienten angeboten.

Für bestimme Langzeitimplantate, wie z.B. tracheo-bronchiale Stents, eignen sich ausschließlich hochmolekulare, also hochviskose Ausgangsmaterialien. Diese führen zu hohen Dehnfestigkeiten von mehr als 500 % bei Spannungen von 10 MPa. Die Härte liegt bei 40 bis 90 Shore A. Dadurch ist es möglich, Gefäßstützen mit hoher Stabilität bei geringer Wandstärke herzustellen.

Die Formgebung dieser Silikonelastomere zu Stents erfolgt entweder für hochviskoses Silikon (HCR High Consisty Rubber) durch Heißpressen oder für Flüssigsilikon LSR (Liquid Silicone Rubber) durch Spritzguss in ein beheiztes Werkzeug. Die Werkzeuge bestehen aus Stabilitätsgründen und zur besseren Wärmeleitung vorwiegend aus Metall. Die Vulkanisation findet bei 120°C bis 180 °C für etwa 10 s pro mm Wandstärke statt. Eine höhere Temperatur reduziert die Vulkanisationszeit.

Bisherige tracheo-bronchiale Stents aus Silikon sind als Serienprodukte in der Medizin etabliert. Es gibt jedoch eine Anzahl von Patienten, die aus bestimmten anatomischen oder medizinischen Gesichtspunkten nicht mit einem Serienprodukt behandelt werden können, sondern die auf eine kurzfristig verfügbare individuelle Gefäßstütze angewiesen sind. Bei einer Stückzahl von eins bietet sich theoretisch die Herstellung über sogenannte additive Verfahren an, da diese Verfahren eine direkte Formgebung ermöglichen und weil keine Werkzeugkosten anfallen.

Der direkte 3D-Druck von LSR-Masse über sogenannte Pastenextruder führt allerdings zu Produkten mit geringer Auflösung. Die Komponenten A und B der LSR-Masse werden meist unter Druck in einem statischen Mischrohr direkt vor dem Austrag gemischt und durch ein 3-achsiges computergesteuertes Portal abgelegt. Die hohe Viskosität verhindert eine feine Auflösung beim Druck. Die einzelnen Schichten sind deutlich im unteren mm-Bereich sichtbar. Die Vulkanisation findet in diesem Fall schichtweise durch Wärmeeintrag über Heißluft oder IR-Strahlung statt.

Ein direkter 3D-Druck von UV-härtbaren Flüssigsilikonen durch Stereolithographie (SLA) ist aufgrund der zu hohen Viskosität nicht möglich. Verfügbare UV-härtbare dünnflüssige Silikone sind nicht biokompatibel bzw. weisen nicht gleichzeitig die erforderlichen mechanischen Kennwerte auf, so dass sie für den 3D-Druck geeignet sind. Weiterhin zeigt die theoretische Betrachtung der Silikonchemie einen Widerspruch zwischen Viskosität und mechanischen Eigenschaften. Polysiloxane mit niedriger Viskosität im unvernetzten Zustand besitzen gleichzeitig geringe mechanische Eigenschaften im vernetzten Zustand aufgrund der kürzeren Präpolymere.

Daher ist es nicht möglich, mittels additiver Fertigung ein biokompatibles medizinisch geeignetes und mechanisch belastbares Implantat mit hoher Oberflächenqualität aus Silikon erzeugen, das zudem wirtschaftlich in der Losgröße eins hergestellt werden kann.

Zum Stand der Technik zählt die Veröffentlichung Sereno, L. et al., "New advances on tracheal stent manufacturing", 6th IFAC Conference on Management and Control of Production and Logistics, The International Federation of Automatic Control, September 11-13, 2013 Fortaleza, Brazil, S.344-349. Es werden verschiedene Herstellungsverfahren zur Herstellung von Silikonstents aus biokompatiblen Silikonen offenbart. Es wurde durch additive Fertigung ein Formwerkzeug aus dem Werkstoff ABS hergestellt. Eingespritztes Silikon wurde für 5 Minuten bei 180°C vulkanisiert. Es wurden keine weiteren Untersuchungen dahingehend vorgenommen, ob die Biokompatibilität bzw. chemische Reinheit des Implantats auch nach dem Kontakt mit dem Werkstoff ABS gegeben ist. US2006/204699 A1 offenbart ein Verfahren zur Herstellung eines Mikrofluidgeräts aus Silikon mittels einer Gussform welche durch ein additives Fertigungsverfahren aus einem polymeren Werkstoff hergestellt wurde.

Der Erfindung liegt daher die Aufgabe zu Grunde, ein Verfahren zur Herstellung von biokompatiblen, medizinisch geeigneten, mechanisch belastbaren Implantaten mit hoher Oberflächenqualität aus Silikon aufzuzeigen, wobei das Verfahren auch bei sehr geringer Stückzahl wirtschaftlich günstiger ist als bekannte Herstellverfahren. Diese Aufgabe wird durch ein Verfahren mit den Merkmalen des Patentanspruchs 1 gelöst.

Vorteilhafte Weiterbildungen der Erfindung sind in den Unteransprüchen enthalten.

Das erfindungsgemäße Verfahren zur Herstellung eines Langzeitimplantats, aus biokompatiblen Silikon wird mit folgenden Schritten durchgeführt:
a) Es wird ein mathematisches 3D-Modell eines herzustellenden Implantats bereitgestellt;
b) Mittels des 3-D-Modells des Implantats wird als Negativ ein 3D-Modell einer Gussform für das Implantat erstellt;
c) Die Gussform wird durch ein additives Fertigungsverfahren aus einem polymeren Werkstoff herstellt;
d) Die Gussform wird mindestens in Bereichen, die mit dem zu vergießenden Silikon in Kontakt kommen, beschichtet;
e) Das Beschichten erfolgt mittels Gasphasenabscheidung eines Beschichtungswerkstoffes aus der Familie der Parylene;
f) Das biokompatible Silikon wird in den Formhohlraum der beschichteten Gussform eingebracht;
g) Die Gussform wird erwärmt, so dass das Silikon vulkanisiert;
h) Das vulkanisierte Implantat wird nach einem Abkühlen aus der Gussform entformt.

Es handelt sich bei dem Implantat um ein Langzeitimplantat. Der Begriff Langzeit bezieht sich auch eine Verweildauer im Körper von mehr als 29 Tagen. Das Implantat ist insbesondere ein Stent.

Das erfindungsgemäße Verfahren bedient sich des indirekten 3D-Drucks. Beim indirekten 3D-Druck wird nicht das Bauteil, d.h. das Implantat additiv hergestellt, sondern eine Gussform, mit welcher das Implantat geformt werden kann. Die Beschränkung des 3D-Druckverfahrens liegt darin, dass ein Prozess und ein Material gefunden werden muss, welches eine ausreichende Stabilität zum Abformen von geeigneten Silikonen aufweist, sowie gleichzeitig eine hohe Druckauflösung besitzt, um glatte Oberflächen zu erzeugen und welches ferner den Temperaturen zur Vulkanisation des Silikons für eine bestimmte Zeit Stand hält. Weiter dürfen beim Formgebungsprozess und bei der Vulkanisation keine Inhaltsstoffe aus der Gussform in das Implantat migrieren. Schließlich muss sich das vulkanisierte Implantat nach der Vulkanisation entformen lassen, ohne durch zu hohe Kräfte beschädigt zu werden.

Das erfindungsgemäße Verfahren basiert darauf, dass zunächst ein mathematisches 3D-Modell eines herzustellenden Implantats bereitgestellt wird. Das 3D-Modell stellt die Eingangsinformationen des erfindungsgemäßen Verfahrens dar. Insbesondere kann das 3D-Modell aus einem biologischen Volumenmodell des Stents abgeleitet werden, das zuvor aus bildgebenden Daten, wie z.B. einer Computertomografie oder einer Magnetresonanztomografie oder anderen 3D-Daten, wie z.B. aus einem 3D-Scan gewonnen wurde.

Die aus den bildgebenden Daten gewonnenen Konturen für das Volumenmodell, insbesondere einen Stent, können zur Erzielung einer therapeutischen Wirkung geändert werden. Hierzu werden medizinische Fachkenntnisse angewendet, die sicherstellen, dass die gewünschte therapeutische Wirkung erzielt werden kann, ohne dabei negative Nebenwirkungen zu erzeugen, z.B. Nekrosen.

Das erfindungsgemäße Verfahren zur Herstellung des Implantats bezieht sich auf das Herstellungsverfahren an sich ausgehend von den bereits zur Verfügung gestellten Daten des zu fertigenden Implantats unabhängig davon, ob und wie diese Daten vorher gewonnen, verändert oder bearbeitet worden sind.

Ausgehend von dem 3D-Modell des Implantats, d.h. ausgehend von der Positivform des Implantats, wird eine virtuelle Negativform in Form eines 3D-Modells einer Gussform für das Implantat erstellt. Die Gussform kann einteilig oder mehrteilig sein, um das Entformen des fertigen Implantats zu ermöglichen. Die Gussform enthält einen Formhohlraum für das herzustellende Implantat. Der Formhohlraum ist mit den für das Gießen von Silikon erforderlichen Anschlüssen, z.B. einen Anguss oder Steiger versehen.

Die eigentliche Gussform wird durch ein additives Fertigungsverfahren hergestellt, d. h. durch ein sogenanntes 3D-Druckverfahren, wie z.B. die Stereolithographie oder das sogenannte Material Jetting. Das Drucken kann mit relativ geringer Schichtdicke erfolgen, so dass eine hohe Druckauflösung und eine sehr gute Oberflächenbeschaffenheit erreichbar ist, die für medizinische Verwendungen und insbesondere für die in Rede stehenden Implantate wesentlich ist. Die Schichtdicke für die additive Fertigung sollte maximal 50 µm betragen und besser 25 µm nicht überschreiten. Es soll als Werkstoff für die Gussform ein hinreichend temperaturstabiles Druckharz verwendet werden. Insbesondere soll das Druckharz nachträglich nachhärtbar sein, um die für das Vulkanisieren des Silikons erforderliche thermische Stabilität zu erreichen und ferner, um die mechanischen Eigenschaften der Gussformen erfüllen zu können. Die Erfindung ermöglicht es, die Gussform aus Polymeren herzustellen, die nicht-biokompatibel sind. Sofern biokompatible Polymere zur Verfügung stehen, ist es mit dem erfindungsgemäßen Verfahren auch möglich, biokompatible Polymere für die Gussform zu verwenden.

Die Gussform aus einem polymeren Werkstoff wird beschichtet. Die Beschichtung soll verhindern, dass Inhaltsstoffe aus der Gussform während des Formgebungsprozesses oder bei der Vulkanisation in das Silikon, bzw. das Implantat, migrieren. Daher werden mindestens die Bereiche, die mit dem zu vergießenden Silikon in Kontakt kommen, beschichtet, insbesondere aber die ganze Gussform. Es soll daher eine Diffusionsbarriere geschaffen werden, die er überhaupt erst ermöglicht, nicht-biokompatible Werkstoffe für die Gussform zu verwenden und daher den Weg zu den additiven Fertigungsverfahren ebnet.

Die Beschichtung erfolgt mittels Gasphasenabscheidung, d. h. mittels eines CVD-Verfahrens. Als Beschichtungswerkstoffe werden Polymere aus der Parylene-Familie angewendet.

Als Parylene werden eine Gruppe inerte, hydrophobe, optisch transparente, polymere Beschichtungsmaterialien bezeichnet. Die CVD-Abscheidung von Parylenen umfasst im Wesentlichen das Verdampfen eines Prepolymers (Dimers) im Vakuum, die anschließende Pyrolyse des verdampften Dimers zu Monomeren und schließlich die Kondensation und Polymerisation der Beschichtung (Parylene) auf der kalten Oberfläche der Gussform. Aufgrund der gasförmigen Abscheidung und hohen Spaltengängigkeit können auch Bereiche und Strukturen erreicht und beschichtet werden, welche mit flüssigkeitsbasierten Verfahren nicht beschichtbar sind, wie z. B. scharfe Ränder und Spitzen oder enge und tiefe Spalten. In einem Arbeitsgang können Beschichtungsdicken von 0,1 bis 10 µm aufgebracht werden. Ab 0,6 µm Schichtdicke sind die Beschichtungen mikroporen- und pinholefrei. Das Beschichten hat zudem den Vorteil, dass die Oberfläche der Gussform in den beschichteten Bereichen geglättet wird. Vorzugsweise liegt die Beschichtung in einem Bereich größer als 1 µm und vorzugsweise in einem Bereich von 1 bis 10 µm, insbesondere in einem Bereich von 3 bis 7 µm.

Nach dem Beschichten ist die Gussform soweit vorbereitet, dass das biokompatible Silikon in den Formhohlraum der beschichteten Form eingebracht werden kann. Dies erfolgt vorzugsweise unter Druck über einen Anguss. Es handelt sich in diesem Fall um eine Spritzgussverfahren unter Verwendung des indirekten 3D-Drucks. Anschließend muss das Silikon vernetzt, d. h. vulkanisiert werden. Hierfür wird die Gussform erwärmt. Das vulkanisierte Implantat wird nach einem Abkühlen aus der Gussform entformt und der weiteren Verwendung zugeführt.

Bevorzugt wird mit diesem Verfahren ein Stent, insbesondere ein tracheo-bronchialer Stent, als Implantat hergestellt. Die Herstellung ist auch dann wirtschaftlich von Vorteil, wenn ein solcher individuell angefertigter tracheo-bronchialer Stent in der Stückzahl eins hergestellt wird. Es muss keine aufwendige Metallspritzgussform hergestellt werden, was zu erheblichen Kosten bei geringen Stückzahlen führen würde.

Da es tracheo-bronchiale Stents als Serienbauteile gibt, liegen Konstruktionskenntnisse der Serienbauteile vor, welche auch die therapeutischen Wirkungen bereits berücksichtigen und so konzipiert sind, dass möglichst wenige negative Nebenwirkungen erzeugt werden. Bei dem erfindungsgemäßen Verfahren kann auf diese bekannten Informationen zurückgegriffen werden, so dass das 3D-Modell des Implantats in einem CAD-System als Negativ in einen virtuellen Block, d.h. eine Gussform hineingelegt werden kann und über bool'sche Operationen abgezogen werden kann. Der so in dem Block entstehende Hohlraum ist der spätere Formhohlraum. Der Block wird anschließend insbesondere mehrteilig gestaltet, um das gegossene Implantat aus der späteren Gussform entnehmen zu können. Hierzu kann die Gussform wenigstens eine erste Werkzeughälfte, wenigstens eine zweite Werkzeughälfte und wenigstens einen Kern aufweisen, wobei all diese Komponenten bei dem erfindungsgemäßen Verfahren additiv gefertigt werden. Hinzu kommen die spritzgusstypischen Module wie Anguss, Entlüftungen, und weitere Funktionsflächen, wie z.B. Zentrierungen und Fixierungen der Werkzeugteile aneinander.

Die einzelnen Komponenten der so konstruierten Gussform werden in einem stereolithographischen Dateiformat aus einem CAD-System exportiert und in einer für den beabsichtigten 3D-Druck geeigneten Slicer-Software für den 3D-Druck vorbereitet. Während dieser Stufe kann auch eine mögliche Schrumpfung der Bauteile als Aufmaß berücksichtigt werden.

Als Werkstoff für die Gussform wird insbesondere ein UV-aushärtbares Harz verwendet. Die Verwendung eines UV-aushärtbaren Harzes ermöglicht es, der Gussform die notwendige Formstabilität und auch die thermische Stabilität für das spätere Vulkanisieren zu geben. Je nach Herstellervorgaben für das UV-aushärtbare Harz kann das UV-Härten (Nachhärten) in mehreren Stufen erfolgen, um die thermische Stabilität der Gussform sicherzustellen. Beispielsweise kann das UV-Härten der Gussform oder eines Teiles der Gussform in wenigstens drei Stufen erfolgen, wobei in einer ersten Stufe eine Temperatur von nicht weniger als 60 °C über eine Haltezeit von mindestens 60 Minuten eingestellt wird. Es schließt sich eine zweite Stufe bei einer Temperatur von nicht weniger als 80 °C über eine Haltezeit von mindestens 60 Minuten an und schließlich eine dritte Stufe mit einer Temperatur von nicht weniger als 120 °C über eine Haltezeit von mindestens 120 Minuten. Vor dem Härten werden die 3D-gedruckten Bauteile von drucktypischen Stützmaterialien befreit und gereinigt. Das Reinigen kann insbesondere durch Waschen in Isopropanol für mindestens 10 Minuten erfolgen und anschließendes Trocknen. Daraufhin beginnt das Nachhärten unter UV-Strahlung und Wärme.

Vorzugsweise wird die so hergestellte Gussform vor der Weiterverarbeitung mittels eines Plasmareinigungsverfahrens gereinigt. Hierbei kann das Sauerstoff-Plasmaätzen zur Anwendung kommen, welches auch gleichzeitig die Haftung des Parylens erhöht. Beispielsweise kann die Gussform im Vakuum für mindestens 3 Minuten mit einem Sauerstoff-Plasma gereinigt werden.

Eine weitere Vorbehandlung kann durch Silanisieren erfolgen. Das Silanisieren ist auch als Silicoterverfahren bekannt. Die vorzugsweise Abscheidung von Silangruppen erfolgt durch eine Abscheidung eines sogenannten Precursers in einem Vakuumprozess. Ein flüssiges Silan wird dabei verdampft und lagert sich auf der Oberfläche des Bauteils durch Kondensation ab, wodurch ein Haftgrund erzeugt wird.

Anschließend wird die eigentliche Beschichtung aufgebracht, wobei dafür ein Polymer aus der Familie der Parylene verwendet wird. Insbesondere wird Parylene C über das CVD-Verfahren abgelagert. Vorzugsweise beträgt die Dicke der Parylene C-Schicht mindestens 1 µm, insbesondere mindestens 5 µm. Die Schichtdicke liegt vorzugsweise in einem Bereich zwischen 1 und 10 µm. Parylene haben die Eigenschaft, dass sie diffusionsdicht sind. Sie bilden einen geschlossenporigen Film. Die Diffusionsbarriere und die geschlossenporige Struktur ermöglichen es erfindungsgemäß, Implantate aus Silikon herzustellen, in die keine schädlichen Stoffe aus der Gussform diffundieren. Parylen C verfügt über die notwendige physikalischen Eigenschaften und weist eine extrem niedrige Durchlässigkeit gegenüber Feuchtigkeit und Gasen auf.

Die so beschichtete Gussform wird anschließend vor der Weiterbearbeitung kontrolliert, gegebenenfalls noch mal gereinigt und Teile der Gussform werden zu der zu der fertigen Gussform zusammengesetzt. Die Montage der mehrteiligen Gussform kann beispielsweise durch Verschrauben der Gussformteile erfolgen, durch welche der Formholraum in den maßgeblichen formgebenden Bereichen druckdicht verschlossen ist. Das Schließen des Formhohlraums, bzw. das Verbinden von Werkzeughälften, kann mittels Schrauben erfolgen. Denkbar ist auch die Werkzeughälften miteinander zu verspannen, beispielsweise in einer Presse.

Anschließend wird unter Druck gemischtes, implantierbares medizinisches Silikon, insbesondere medizinisch zugelassenes platinkatalysiertes 2K-Silikon verwendet, z.B. Nusil MED-4870 (Nusil ist eine eingetrage Marke der Nusil Techology, LLC) in den Formhohlraum der Gussform eingebracht. Das Einbringen des Silikons, insbesondere das Einspritzen unter Druck kann auch in ein vorgeheiztes Werkzeug erfolgen. Der Füllvorgang ist abgeschlossen, wenn aus wenigstens einer Entlüftungsöffnung, die an dem Formhohlraum angeschlossen ist, Silikon auszutreten beginnt. Das so eingebrachte Silikon wird anschließend vulkanisiert, insbesondere bei 120 °C bis 180 °C für 10 Sekunden je Millimeter Wanddicke des Implantats. Vorzugsweise wird die Gussform, bzw. der vom Hohlraum der Gussform, auf eine Temperatur von mindestens 140 °C für mindestens 10 Sekunden je Millimeter Wanddicke des Implantats erwärmt. Hierzu kann die gesamte Gussform in einen Ofen gelegt werden und so lange bei 140 °C erwärmt werden, bis die Temperatur im Inneren 140 °C beträgt. Die Temperatur im Formhohlraum kann über wenigstens einen in dem wenigstens einen Kern angeordneten Temperaturfühler gemessen werden. Da der Kern von dem Werkstück umgeben ist, lässt die Temperatur im Kern eine Aussage über die Temperatur im Formhohlraum zu. Der bevorzugte Wanddickenbereich für Stents beträgt 0,5 mm bis 3 mm.

Das Erwärmen der Gussform zum Vulkanisieren des Silikons kann mittels Heißluft und/oder IR-Strahlung erfolgen. Entscheidend ist, dass die Vulkanisation solange erfolgt, bis das gesamte Silikon, d. h. die sämtlichen Dickenbereiche vulkanisiert sind.

Die Gussform wird nach dem Abkühlen geöffnet, so dass das Implantat entformt werden kann. Anschließend kann das Silikonbauteil mechanisch nachbehandelt werden, wie beispielsweise dadurch, dass Angusskegel, Steiger, anhaftende Schwimmhäute und sonstige unerwünschte Abschnitte des Implantats entfernt werden. Das Implantat kann anschließend gereinigt werden und optional beschichtet werden, beispielsweise durch Dipcoaten zur Erzielung der gewünschten Oberflächenqualität. Die formgebenden Fertigungsschritte des Implantats sind nun abgeschlossen.

Für die weitere Verwendung wird das Implantat vorzugsweise in eine geeignete, heißsiegelfähige Folie verpackt, die eine Sterilisation zulässt und eine Kontamination ausschließt. Insbesondere handelt es sich hierbei um einen Beutel aus Tyvek (Tyvek ist eine eingetragene Marke der Firma E.I. Du Pont de Nemours and Co.), in den das Implantat verpackt und heißgesiegelt wird. Es schließt sich ein Sterilisationsprozess an, wobei die Sterilisation z.B. mittels Dampf, ETO oder Wasserstoffperoxid-Plasma erfolgen kann.

Mit dem erfindungsgemäßen Verfahren ist es möglich, biokompatible, medizinisch geeignete und mechanisch belastbare Implantate mit hoher Oberflächenqualität aus Silikon herzustellen, die auch bei sehr geringer Stückzahl wirtschaftlich günstiger herstellbar sind als mit anderen bekannten Herstellverfahren. Insbesondere ist es möglich, die Technologie der Stereolithographie oder des Material Jettings zu nutzen ohne die Biokompatibilität zu gefährden. Die besagten Implantate erfüllen die Erfordernisse der physikalisch-chemischen Prüfungen und Analysen von gelösten Stoffen und Substanzen gemäß der Norm ISO 10993 und halten Grenzwerte von herauslösbaren Substanzen ein. Es wurde ein Prozess und die Verwendung von Materialien aufgezeigt, welche eine ausreichende Stabilität zum Abformen von Flüssigsilikonen ermöglichen. Das fertige Erzeugnis besitzt aufgrund der hohen Druckauflösung eine glatte Oberfläche, wobei Oberflächenunebenheiten durch die nachträgliche CVD-Beschichtungen mittels eines Parylens weiter reduziert worden sind. Zudem werden durch die Beschichtungen Migrationen von Inhaltsstoffen aus der Gussform in das Bauteil verhindert, so dass die besagte Norm eingehalten werden kann.

## Patentansprüche

1. Verfahren zur Herstellung eines Implantats aus einem biokompatiblen Silikon mit folgenden Schritten:
a) Es wird ein mathematisches 3D-Modell eines herzustellenden Implantats bereitgestellt;
b) Mittels des 3-D-Modells des Implantats wird als Negativ ein 3D-Modell einer Gussform für das Implantat erstellt;
c) Die Gussform wird durch ein additives Fertigungsverfahren aus einem polymeren Werkstoff hergestellt;
d) Die Gussform wird mindestens in Bereichen, die mit dem zu vergießenden Silikon in Kontakt kommen, beschichtet;
e) Das Beschichten erfolgt mittels Gasphasenabscheidung eines Beschichtungswerkstoffes aus der Familie der Parylene;
f) Das biokompatible Silikon wird in den Formhohlraum der beschichteten Gussform eingebracht, wobei als Werkstoff für das Implantat platinkatalysierte 2-komponentige warmhärtende Silikone verwendet werden für eine Verweildauer des Implantats im Körper eines Patienten von mehr als 29 Tagen;
g) Die Gussform wird erwärmt, so dass das Silikon vulkanisiert;
h) Das vulkanisierte Implantat wird nach einem Abkühlen aus der Gussform entformt.

2. Verfahren nach Anspruch 1, **dadurch gekennzeichnet, dass** die Gussform aus einem nicht-biokompatiblen Werkstoff hergestellt wird.

3. Verfahren nach Anspruch 1 oder 2, **dadurch gekennzeichnet, dass** mittels des Verfahrens ein Stent, insbesondere ein tracheo-bronchialer Stent, als Implantat hergestellt wird.

4. Verfahren nach einem der Ansprüche 1 bis 3, **dadurch gekennzeichnet, dass** das 3D-Modell für die Gussform mehrteilig gestaltet wird, um das gegossene Implantat aus der Gussform entnehmen zu können.

5. Verfahren nach Anspruch 4, **dadurch gekennzeichnet, dass** für die Gussform wenigstens eine erste Werkzeughälfte, wenigstens eine zweite Werkzeughälfte und wenigstens ein Kern additiv gefertigt werden.

6. Verfahren nach einem der Ansprüche 1 bis 5, **dadurch gekennzeichnet, dass** als Werkstoff für die Gussform ein UV-aushärtbares Harz verwendet wird, wobei die Gussform oder deren Teile nach dem 3D-Drucken gereinigt werden und in einer oder mehreren Temperaturstufen UV-gehärtet werden.

7. Verfahren nach Anspruch 6, **dadurch gekennzeichnet, dass** das UV-Härten der Gussform in wenigstens drei Stufen erfolgt, wobei in einer ersten Stufe eine Temperatur von nicht weniger als 60°C über eine Haltezeit von mindestens 60 min eingestellt wird, wobei in einer sich anschließenden zweiten Stufe eine Temperatur von nicht weniger als 80°C über eine Haltezeit von mindestens 60 min eingestellt wird, und wobei in einer sich anschließenden dritten Stufe eine Temperatur von nicht weniger als 120°C über eine Haltezeit von mindestens 120 min eingestellt wird.

8. Verfahren nach einem der Ansprüche 1 bis 7, **dadurch gekennzeichnet, dass** die Gussform vor dem Beschichten mit einem Plasmareinigungsverfahren gereinigt wird.

9. Verfahren nach einem der Ansprüche 1 bis 8, **dadurch gekennzeichnet, dass** die gereinigte Gussform vor dem Beschichten silanisiert wird.

10. Verfahren nach einem der Ansprüche 1 bis 9, **dadurch gekennzeichnet, dass** die Beschichtung mit einer Dicke von mindestens 1 µm aufgebracht wird.

11. Verfahren nach einem der Ansprüche 1 bis 10, **dadurch gekennzeichnet, dass** die Vulkanisation bei 120°C bis 180 C° für min 10 sec je mm Wanddicke des Implantats erfolgt.

12. Verfahren nach einem der Ansprüche 1 bis 11, **dadurch gekennzeichnet, dass** die Gussform nach dem Einbringen des Silikons in die Gussform erwärmt wird, bis in einem Formhohlraum der Gussform eine Temperatur von mindestens 140°C für min 10 sec je mm Wanddicke des Implantats erreicht wird.

13. Verfahren nach einem der Ansprüche 5 bis 12, **dadurch gekennzeichnet, dass** die Temperatur im Formhohlraum über wenigstens einen in dem wenigstens einen Kern angeordneten Temperaturfühler gemessen wird.

14. Verfahren nach einem der Ansprüche 1 bis 13, **dadurch gekennzeichnet, dass** das Erwärmen der Gussform zum Vulkanisieren des Silikons mittels Heißluft und/oder IR-Strahlung erfolgt.

15. Verfahren nach einem der Ansprüche 1 bis 14, **dadurch gekennzeichnet, dass** das fertige Implantat in eine heißsiegelfähige Folie eingelegt wird, die eine Sterilisation zulässt und eine Kontamination ausschließt.

16. Verfahren nach einem der Ansprüche 1 bis 15, **dadurch gekennzeichnet, dass** das fertige Implantat mittels Dampf, ETO oder H202-Plasma sterilisiert wird.

## Claims

1. Method for producing an implant made from a biocompatible silicone having the following steps:
a) a mathematical 3D model of an implant to be produced is provided;
b) by means of the 3D model of the implant, a 3D model of a pouring shape for the implant is produced as a negative;
c) the pouring shape is produced by means of an additive manufacturing method from a polymeric material;
d) the pouring shape is coated at least in areas which come into contact with the silicone to be poured;
e) the coating is carried out by means of the vapour phase deposition of a coating material from the family of the parylenes;
f) the biocompatible silicone is introduced into the shape cavity of the coated pouring shape, wherein as a material for the implant, platinum catalysed 2-component thermosetting silicones are used for a stay time of the implant in the body of the patient of more than 29 days;
g) the pouring shape is heated so that the silicone vulcanises;
h) the vulcanised implant is removed from the pouring shape after cooling.

2. Method according to claim 1, **characterised in that** the pouring shape is produced from a material which is not biocompatible.

3. Method according to claim 1 or 2, **characterised in that** by means of the method a stent, in particular a tracheobronchial stent, is produced as an implant.

4. Method according to any of claims 1 to 3, **characterised in that** the 3D model for the pouring shape is designed in multiple parts, in order to be able to remove the poured implant from the pouring shape.

5. Method according to claim 4, **characterised in that** for the pouring shape at least one first tool half, at least one second tool half and at least one core are additively produced.

6. Method according to any of claims 1 to 5, **characterised in that** as a material for the pouring shape a UV-curable resin is used, wherein the pouring shape or its parts are cleaned after the 3D printing and are UV-cured in one or several temperature stages.

7. Method according to claim 6, **characterised in that** the UV-curing of the pouring shape takes place in at least three stages, wherein in a first stage a temperature of not less and 60°C is set for a holding time of at least 60 minutes, wherein in a subsequent second stage a temperature of not less than 80°C is set for a holding time of at least 60 minutes, and wherein in a subsequent third stage a temperature of not less than 120°C is set for a holding time of at least 120 minutes.

8. Method according to any of claims 1 to 7, **characterised in that** the pouring shape is cleaned prior to the coating with a plasma cleaning method.

9. Method according to any of claims 1 to 8, **characterised in that** the cleaned pouring shape is silanated prior to the coating.

10. Method according to any of claims 1 to 9, **characterised in that** the coating is applied with a thickness of at least 1 µm.

11. Method according to any of claims 1 to 10, **characterised in that** the vulcanisation takes place at 120°C to 180°C for at least 10 seconds per millimetre wall thickness of the implant.

12. Method according to any of claims 1 to 11, **characterised in that** the pouring shape is heated after the introduction of the silicone into the pouring shape until in a shape cavity of the pouring shape a temperature of at least 140°C for at least 10 seconds per millimetre wall thickness of the implant is achieved.

13. Method according to any of claims 5 to 12, **characterised in that** the temperature in the shape cavity is measured by way of at least one temperature sensor arranged in the at least one core.

14. Method according to any of claims 1 to 13, **characterised in that** the heating of the pouring shape for vulcanising the silicone takes place by means of hot air and/or IRradiation.

15. Method according to any of claims 1 to 14, **characterised in that** the finished implants is laid into a heat-sealable film which permits sterilisation and precludes contamination.

16. Method according to any of claims 1 to 15, **characterised in that** the finished implant is sterilised by means of steam, ETO or H202 plasma.

## Revendications

1. Procédé de fabrication d'un implant en silicone biocompatible, comprenant les étapes suivantes :
a) un modèle mathématique 3D d'un implant à fabriquer est fourni ;
b) un modèle 3D d'un moule de coulée pour l'implant est créé en négatif à l'aide du modèle 3D de l'implant ;
c) le moule de coulée est fabriqué à partir d'un matériau polymère par un procédé de fabrication additive ;
d) le moule de coulée est revêtu au moins dans les zones qui entrent en contact avec le silicone à couler ;
e) le revêtement est réalisé par dépôt en phase vapeur d'un matériau de revêtement de la famille des parylènes ;
f) le silicone biocompatible est introduit dans la cavité de moulage du moule de coulée revêtu, dans lequel on utilise comme matériau pour l'implant des silicones thermodurcissables à deux composants catalysés par du platine pour une durée de maintien de l'implant dans le corps d'un patient supérieure à 29 jours ;
g) le moule de coulée est chauffé de sorte que le silicone se vulcanise ;
h) l'implant vulcanisé est démoulé après un refroidissement du moule de coulée.

2. Procédé selon la revendication 1, **caractérisé en ce que** le moule de coulée est fabriqué à partir d'un matériau non biocompatible.

3. Procédé selon la revendication 1 ou 2, **caractérisé en ce qu'**un stent, en particulier un stent trachéo-bronchique, est fabriqué comme implant au moyen du procédé.

4. Procédé selon l'une quelconque des revendications 1 à 3, **caractérisé en ce que** le modèle 3D pour le moule de coulée est conçu en plusieurs parties de sorte que l'implant coulé puisse être retiré du moule de coulée.

5. Procédé selon la revendication 4, **caractérisé en ce que**, pour le moule de coulée, au moins une première moitié d'outil, au moins une seconde moitié d'outil et au moins un noyau sont fabriqués par fabrication additive.

6. Procédé selon l'une quelconque des revendications 1 à 5, **caractérisé en ce que** l'on utilise comme matériau pour le moule de coulée une résine durcissable par UV, le moule de coulée ou ses parties étant nettoyés après l'impression 3D et durcis par UV en une ou plusieurs étapes de température.

7. Procédé selon la revendication 6, **caractérisé en ce que** le durcissement par UV du moule de coulée est effectué en au moins trois étapes, dans lequel, lors d'une première étape, une température non inférieure à 60 °C est réglée pendant un temps de maintien d'au moins 60 minutes, dans lequel, lors d'une deuxième étape consécutive, une température non inférieure à 80 °C est réglée pendant un temps de maintien d'au moins 60 minutes, et dans lequel, lors d'une troisième étape consécutive, une température non inférieure à 120 °C est réglée pendant un temps de maintien d'au moins 120 minutes.

8. Procédé selon l'une quelconque des revendications 1 à 7, **caractérisé en ce que** le moule de coulée est nettoyé par un procédé de nettoyage au plasma avant le revêtement.

9. Procédé selon l'une quelconque des revendications 1 à 8, **caractérisé en ce que** le moule de coulée nettoyé est silanisé avant le revêtement.

10. Procédé selon l'une quelconque des revendications 1 à 9, **caractérisé en ce que** le revêtement est appliqué avec une épaisseur d'au moins 1 µm.

11. Procédé selon l'une quelconque des revendications 1 à 10, **caractérisé en ce que** la vulcanisation est effectuée à une température de 120 °C à 180 °C pendant au moins 10 secondes par mm d'épaisseur de paroi de l'implant.

12. Procédé selon l'une quelconque des revendications 1 à 11, **caractérisé en ce que** le moule de coulée est chauffé après l'introduction du silicone dans le moule de coulée jusqu'à ce qu'une température d'au moins 140 °C soit atteinte dans une cavité de moulage du moule de coulée pendant au moins 10 secondes par mm d'épaisseur de paroi de l'implant.

13. Procédé selon l'une quelconque des revendications 5 à 12, **caractérisé en ce que** la température dans la cavité de moulage est mesurée par au moins un capteur de température agencé dans l'au moins un noyau.

14. Procédé selon l'une quelconque des revendications 1 à 13, **caractérisé en ce que** le chauffage du moule de coulée pour la vulcanisation du silicone est effectué au moyen d'air chaud et/ou d'un rayonnement IR.

15. Procédé selon l'une quelconque des revendications 1 à 14, **caractérisé en ce que** l'implant fini est placé dans un film thermosoudable qui permet une stérilisation et exclut une contamination.

16. Procédé selon l'une quelconque des revendications 1 à 15, **caractérisé en ce que** l'implant fini est stérilisé à la vapeur, à l'ETO ou au plasma H202.
